# EUROPEAN PATENT APPLICATION

(11) **EP 4 775 565 A1**
(43) Date of publication of application: **15.07.2026**
(21) Application number: 24862733.3
(22) Date of filing: 30.08.2024
(51) Int. Cl.: C07C 209/84, B01J 39/05, B01J 39/07, B01J 39/19, B01J 47/02, B01J 47/016, C02F 1/42, C07B 63/02, C07C 211/63

(54) **METHOD FOR PRODUCING PURIFIED QUATERNARY AMMONIUM COMPOUND AQUEOUS SOLUTION**

(30) Priority: 04.09.2023 JP 2023142893
(71) Applicant: TOKUYAMA CORPORATION, Yamaguchi 745-8648 (JP)
(72) Inventor: INOUE, Hiroshi, Shunan-shi, Yamaguchi 745-8648 (JP); KAWABATA, Yuichiro, Shunan-shi, Yamaguchi 745-8648 (JP)
(74) Representative: Watermeyer, Nicholas
(86) International application number: PCT/JP2024/031288
(87) International publication number: WO 2025/053081

(57) **Abstract**

Provided is a method for producing a quaternary ammonium compound aqueous solution, said method being characterized in that: the quaternary ammonium compound aqueous solution contains not more than 1,000 ppm of organic impurities that have a molecular weight of not less than 1,000 g/mole; and a raw quaternary ammonium compound aqueous solution that contains specific quaternary ammonium ions and that contains metal impurities is brought into contact with a cation exchange resin which has a crosslinking degree of not less than 6 and counter ions of which are non-metal ions.

## Description

### Technical Field

The present invention relates to a method for producing a purified quaternary ammonium compound aqueous solution and specifically relates to the production method for reducing the content of a metal impurity from a crude quaternary ammonium compound aqueous solution containing the metal impurity as an impurity.

### Background Art

Quaternary ammonium compounds are used in phase transfer catalysts, surfactants, disinfectants, and the like. In particular, quaternary ammonium hydroxide compounds represented by tetramethylammonium hydroxide are used as a kind of strong organic base and utilized in pH adjusters, for washing, etching, developers, and the like in the form of an aqueous solution in semiconductor manufacturing. In such treatment agent applications related to semiconductors, as integration advances, a metal impurity causes a defect when remaining on the surface of an electronic device. Thus, it is necessary to decrease the impurity as much as possible. Specifically, it is desired to reduce the content of metal impurities, such as Na, K, Li, Ca, Mg, and Sr, to 100 ppt or less in terms of the total content of these.

A quaternary ammonium compound aqueous solution is usually produced by a synthesis method with a tertiary amine and an alkyl salt, an electrolytic decomposition method with an electrolytic cell using an ion-exchange membrane, or the like. For example, a quaternary ammonium hydroxide aqueous solution is synthesized by subjecting a quaternary ammonium salt aqueous solution as a raw material to electrolysis or the like. In the quaternary ammonium hydroxide aqueous solution thus synthesized (or the quaternary ammonium salt aqueous solution as a raw material for preparing this), the metal impurities are contained in high levels of several hundred ppt or more, which is not satisfactory in terms of the defect prevention effect in semiconductor applications.

As a method for purifying such a crude quaternary ammonium compound aqueous solution, filter treatment is widely used. Filter treatment provides an excellent effect of decreasing particulate impurities even though they are the metal impurities, but on the other hand, unfortunately, exhibits almost no effect of removing a dissociated ion because of a limit of a pore diameter.

For removal of a metal ion from a quaternary ammonium compound aqueous solution, adsorption treatment with a cation-exchange resin is considered effective. For example, Patent Document 1 shows that such treatment is applied to a quaternary ammonium hydroxide compound aqueous solution synthesized by the electrolysis method described above, and the concentrations of metal ions, such as a Na ion and a Ca ion, are each decreased to 1 ppb or less (see [0016] Reference Example 1 and the like).

In addition, it is also known that adsorption treatment of a crude quaternary ammonium hydroxide compound aqueous solution (in the present invention, the crude quaternary ammonium hydroxide compound aqueous solution is a quaternary ammonium compound aqueous solution containing 1000 ppm or less of an organic impurity with a molecular weight of 1000 g/mol or more and containing a metal impurity) with a cation-exchange resin is also applied for the purpose of recovering the quaternary ammonium hydroxide compound component from a waste liquid when the quaternary ammonium hydroxide compound aqueous solution is used in a photoresist developer for manufacturing a semiconductor (e.g., Patent Document 2). Here, with reference to Patent Document 3 (lower right column in page 3), a strongly acidic cation-exchange resin product shown as a representative example of the cation-exchange resin and used in Example 1 in Patent Document 2 is described as a resin with a degree of cross-linking of 2 to 10%.

### Prior Art Documents

### Patent Document

Patent Document 1: JP H06-025880 A
Patent Document 2: JP 2003-190949 A
Patent Document 3: JP H01-098617 A

### Summary of Invention

### Technical Problem

Also in the adsorption treatment for the photoresist developer waste liquid with a cation-exchange resin, the effect of decreasing a metal ion has not been sufficiently satisfactory in many cases, either. Furthermore, a study by the present inventors have observed a phenomenon in which even though the effect of decreasing a metal ion is exhibited to a certain high degree at the initial stage of the treatment by selecting the cation-exchange resin in this case, the decreasing effect rapidly deteriorates when the purification is continued and the treatment amount increases. For this reason, the knowledge of removing a metal ion in the case where a developer waste liquid is a liquid to be treated has not been able to be taken into consideration for the purification of the quaternary ammonium compound aqueous solution prepared by the synthesis described above (in other words, the quaternary ammonium compound aqueous solution in a state of low organic impurity content), which requires stable production for a long time. In addition, decreasing a metal ion from a quaternary ammonium compound aqueous solution with a cation-exchange resin is known, but neither technique for decreasing metal content to the order of several to several hundred ppt nor technique for long-term use has been known.

In view of the above background, an object of the present invention is to provide a method that can maintain its removal effect at a high level even though removal of a metal ion from a quaternary ammonium compound aqueous solution with low organic impurity content, such as those prepared by synthesis, is continued in a predetermined treatment amount.

### Solution to Problem

As a result of diligent studies to achieve the above object, the present inventors have found that the above problems can be solved by bringing a cation-exchange resin with a specific degree of cross-linking into contact with the liquid to be treated and completed the present invention.

That is, the present invention provides a method for producing a purified quaternary ammonium compound aqueous solution. The method includes bringing a crude quaternary ammonium compound aqueous solution into contact with a cation-exchange resin with a degree of cross-linking of 6 or more in which a counter ion is a non-metal ion type. The crude quaternary ammonium compound aqueous solution contains: 1000 ppm or less of an organic impurity with a molecular weight of 1000 g/mol or more; and a metal impurity. Here, in the present invention, the purified quaternary ammonium compound aqueous solution is a quaternary ammonium compound aqueous solution prepared by bringing the crude quaternary ammonium hydroxide compound aqueous solution into contact with the cation-exchange resin to decrease the metal ion.

### Effects of Invention

According to the present invention, a metal ion contained in a crude quaternary ammonium compound aqueous solution with low organic impurity content, such as those prepared by synthesis, can be adsorbed and removed by an excellent decreasing effect. In addition, this excellent decreasing effect can be maintained at a high level even though the treatment amount of the crude quaternary ammonium compound aqueous solution to a large quantity. Thus, the method according to the present invention is extremely useful as an industrial method for producing a purified quaternary ammonium compound aqueous solution. In particular, the method according to the present invention is useful as a method for producing a quaternary ammonium compound having a specific alkyl group.

### Brief Description of Drawings

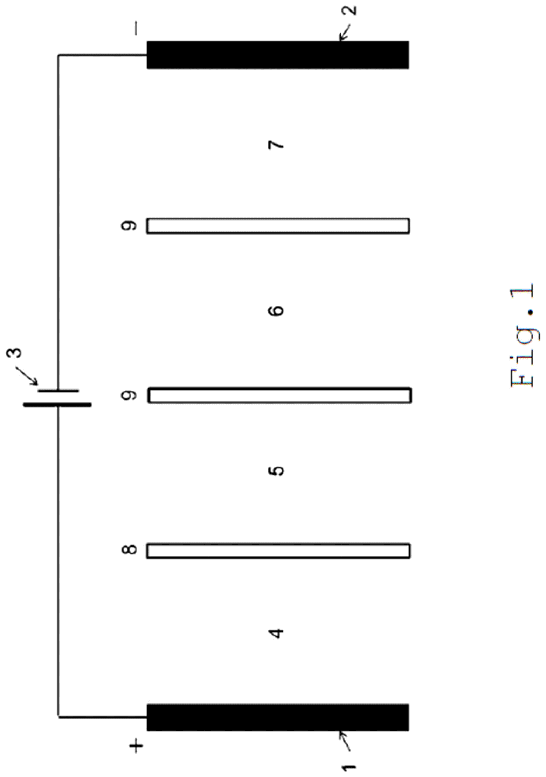

FIG. 1 is a schematic diagram of an electrolytic cell used for producing a quaternary ammonium compound.

### Description of Embodiments

In the present invention, a crude quaternary ammonium compound aqueous solution, which is a liquid to be treated to be brought into contact with a cation-exchange resin, has such a cleanliness that the concentration of an organic impurity with a molecular weight of 1000 g/mol or more is 1000 ppm or less. The impurity is mixed in from a raw material to be used, a contact member of a reaction vessel, a pipe, or a container, and the like. Using a high-purity raw material and appropriately selecting a contact member makes it possible to prepare a quaternary ammonium compound aqueous solution satisfying the requirement for the cleanliness in terms of the organic impurity. In contrast, when it comes to a photoresist developer waste liquid discharged in semiconductor manufacturing, this liquid is also the same type of quaternary ammonium compound aqueous solution but contains organic impurities with large molecular weights (usually such as a novolak resin derivative and/or its decomposition products) derived from the photoresist in various ways. Thus, this does not satisfy the requirement for the cleanliness in terms of the organic impurity. In addition, some organic impurities with large molecular weights can be decreased by neutralizing the photoresist to make it insoluble, but other various organic substances are contained. This does not satisfy the requirement for the cleanliness in terms of the organic impurity, either.

Furthermore, among the organic impurities contained in the crude quaternary ammonium compound aqueous solution, those with a molecular weight of less than 1000 g/mol hardly affect the effect of removing a metal ion with the cation-exchange resin. Thus, the crude quaternary ammonium compound aqueous solution can contain an organic impurity with such a small molecular weight, but the content thereof is preferably 3 mass% or less. Such a low-molecular-weight organic impurity is considered to mix in from an unreacted component used in the synthesis process of the quaternary ammonium compound aqueous solution, an impurity, and/or the like, and examples include an organic solvent, such as methanol and ethanol, and a surfactant.

The crude quaternary ammonium compound aqueous solution contains the organic impurity with a molecular weight of 1000 g/mol or more in an amount more preferably of 500 ppm or less and particularly preferably of 100 ppm or less.

Measurement of the organic impurity in the crude quaternary ammonium compound aqueous solution is performed by the following procedure. First, a sample of the crude quaternary ammonium compound aqueous solution is injected into a gel permeation chromatography (GPC) column to separate and recover a liquid containing 1000 g/mol or more of an organic impurity. Then, for two liquids, the recovered liquid and an eluent used for the separation by GPC, total organic carbon (TOC) concentrations are measured with a TOC meter. The TOC concentration of the eluent alone is subtracted from the TOC concentration of the recovered liquid, the resulting difference is multiplied by the dilution factor of the crude quaternary ammonium compound aqueous solution in the recovered liquid, and the resulting product is taken as the concentration of the organic impurity in the crude quaternary ammonium compound aqueous solution. When the amount of the liquid recovered after passing through a GPC column is small and the measurement with a TOC meter is difficult or when the TOC concentration is too low, the concentration of the organic impurity can be measured by taking a measure, such as recovering the liquid multiple times or concentrating the liquid.

In the present invention, to the quaternary ammonium compound, a hydroxide or a salt of quaternary ammonium can be applied without limitation. Examples of the quaternary ammonium salt include a halide salt such as a chloride salt or a bromide salt, and a carbonate (including a bicarbonate, a carbonate, and a mixture of these). The quaternary ammonium ion is represented by Formula (1) below, 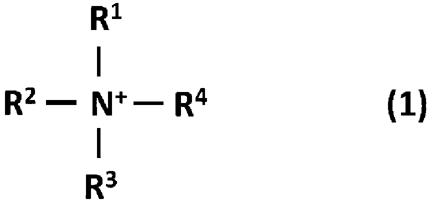 where R¹, R², R³, and R⁴ are each independently an alkyl group having carbon number from 1 to 16, with the proviso that the carbon number of one or more alkyl groups of R¹, R², R³, and R⁴ is from 2 to 16.

In Formula (1) above, all of R¹, R², R³, and R⁴ are optionally identical alkyl groups. For example, all of R¹, R², R³, and R⁴ are optionally ethyl, propyl, or butyl.

However, in Formula (1) above, all of R¹, R², R³, and R⁴ are preferably not all the same alkyl group. In other words, the quaternary ammonium ion is preferably a quaternary ammonium ion represented by (i) to (iii) below:
(i) a quaternary ammonium ion in which any three of R¹, R², R³, and R⁴ are identical alkyl groups, and the remaining one is an alkyl group different from them;
(ii) a quaternary ammonium ion in which any two of R¹, R², R³, and R⁴ are identical alkyl groups and the remaining two are alkyl groups different from them (the remaining two are optionally identical or different); or
(iii) a quaternary ammonium ion in which all of R¹, R², R³, and R⁴ are different alkyl groups.

In the case of (i) above, examples include a quaternary ammonium ion in which any three of R¹, R², R³, and R⁴ are identical alkyl groups having carbon number from 1 to 16, the remaining one is an alkyl group having carbon number from 1 to 16, the first three and the remaining one are different alkyl groups, and either the first three or the remaining one has carbon number from 2 to 16. It is preferred that three of R¹, R², R³, and R⁴ in Formula (1) above be identical groups, and the remaining one be an alkyl group that has carbon number from 2 to 16 and is different from the three identical groups. In this case, the quaternary ammonium ion represented by Formula (1) has two types of alkyl groups. In this aspect, a quaternary ammonium ion in which any three of R¹, R², R³, and R⁴ are methyl groups, and the remaining one alkyl group has carbon number from 2 to 16 is preferred. Among them, a quaternary ammonium ion in which the remaining one alkyl group has carbon number of 2, 3, or 4 is preferred.

In the case of (ii) above, examples include a quaternary ammonium ion in which any two of R¹, R², R³, and R⁴ are identical alkyl groups having carbon number from 1 to 16, the remaining two are alkyl groups that have carbon number from 1 to 16 and are different from the first two, the remaining two are identical or different alkyl groups, and either the first two or the remaining two have carbon number from 2 to 16. In this case, the quaternary ammonium ion represented by Formula (1) has two or three types of alkyl groups.

In the case of (iii) above, examples include a quaternary ammonium ion in which all of R¹, R², R³, and R⁴ are different alkyl groups and at least one of these has carbon number from 2 to 16. In this case, the quaternary ammonium ion represented by Formula (1) has four types of alkyl groups.

Specific examples of the quaternary ammonium compound include hydroxides, such as tetraethylammonium hydroxide, tetrapropylammonium hydroxide, tetrabutylammonium hydroxide, ethyltrimethylammonium hydroxide, propyltrimethylammonium hydroxide, and butyltrimethylammonium hydroxide; chloride salts, such as tetraethylammonium chloride, tetrapropylammonium chloride, tetrabutylammonium chloride, ethyltrimethylammonium chloride, propyltrimethylammonium chloride, and butyltrimethylammonium chloride; bromide salts, such as tetraethylammonium bromide, tetrapropylammonium bromide, tetrabutylammonium bromide, ethyltrimethylammonium bromide, propyltrimethylammonium bromide, and butyltrimethylammonium bromide; carbonate salts, such as tetraethylammonium carbonate, tetrapropylammonium carbonate, tetrabutylammonium carbonate, ethyltrimethylammonium carbonate, propyltrimethylammonium carbonate, and butyltrimethylammonium carbonate. In particular, the quaternary ammonium compound is preferably a quaternary ammonium hydroxide, a quaternary ammonium halide, or a quaternary ammonium carbonate. Furthermore, one or more selected from the group consisting of ethyltrimethylammonium hydroxide, propyltrimethylammonium hydroxide, butyltrimethylammonium hydroxide, ethyltrimethylammonium chloride, propyltrimethylammonium chloride, butyltrimethylammonium chloride, ethyltrimethylammonium bromide, propyltrimethylammonium bromide, butyltrimethylammonium bromide, ethyltrimethylammonium carbonate, propyltrimethylammonium carbonate, and butyltrimethylammonium carbonate are suitably used. Moreover, one or more selected from the group consisting of ethyltrimethylammonium hydroxide, ethyltrimethylammonium chloride, ethyltrimethylammonium bromide, and ethyltrimethylammonium carbonate are suitable. A single type of these quaternary ammonium compounds can be used or a mixture of a plurality of types thereof can be used. In addition, in the present invention, specific examples of the crude quaternary ammonium compound are the same as the specific examples described for the quaternary ammonium compound.

The concentration of the quaternary ammonium compound is not particularly limited. However, the adsorption treatment performed at high concentration usually needs only a small liquid flow amount and eliminates the need for a concentration operation in a later step. Thus, the concentration is preferably 10 mass% or more and more preferably 20 mass% or more. On the other hand, the quaternary ammonium compound at too high a concentration crystallizes or increases the viscosity. Thus, the concentration is preferably 70 mass% or less and more preferably 60 mass% or less. If the quaternary ammonium compound crystallizes at high concentration, the adsorption treatment is performed in a range below that concentration. In addition, in the present invention, the concentration of the quaternary ammonium compound in the aqueous solution can be measured by an ion chromatography method or a potentiometric titration method.

The water component constituting the crude quaternary ammonium compound aqueous solution is preferably as clean as possible and more preferably ultrapure water.

These crude quaternary ammonium compound aqueous solutions contain a metal impurity usually in an amount of 1000 ppt or more or more than 100 ppt even in the case of containing only a small amount. Even the content of such a degree is not satisfactory from the viewpoint of defect prevention when these solutions are used in the semiconductor applications, and purification treatment according to the present invention needs to be applied.

Here, in the present invention, the metal impurity is specifically an element composed of Na, K, Li, Ca, Mg, and Sr, and its content is evaluated by the total content of these. In the present invention, the content of the metal impurity in the quaternary ammonium compound aqueous solution refers to a value analyzed with a high-frequency inductively coupled plasma mass spectrometer (ICP-MS).

The crude quaternary ammonium compound aqueous solution of any origin can be applied as long as the requirements for each content of the organic impurity and the metal impurity are satisfied. Usually, the crude quaternary ammonium compound aqueous solution is preferably a quaternary ammonium hydroxide aqueous solution prepared by synthesis by electrolysis or the like or a quaternary ammonium salt aqueous solution used as a raw material for carrying out the synthesis. Here, the production of the quaternary ammonium hydroxide aqueous solution by electrolysis commonly uses an electrolytic cell in which at least one cation-exchange membrane is placed between an anode and a cathode and which is provided with a raw material chamber for supplying the quaternary ammonium salt aqueous solution as a raw material and a base chamber (cathode chamber) in which the quaternary ammonium hydroxide aqueous solution is produced. In this production method, a quaternary ammonium ion passes through the cation-exchange membrane placed on the cathode side, and a quaternary ammonium hydroxide aqueous solution is produced in the base chamber. In addition, an electrolytic cell in which a plurality of membranes from a cation-exchange membrane, an anion-exchange membrane, and a bipolar membrane (a membrane produced by laminating a cation-exchange membrane and an anion-exchange membrane) is provided has been proposed and can be applied to any electrolytic cell.

In the present invention, the crude quaternary ammonium compound aqueous solution is brought into contact with a cation-exchange resin to adsorb and remove an impurity metal ion to the cation-exchange resin, and the content of the metal impurity is reduced. The crude quaternary ammonium compound aqueous solution exhibits neutrality to basicity, thus the cation-exchange resin can be well used even though the cation-exchange resin is either a strongly acidic cation-exchange resin in which the cation-exchange group is a sulfonic acid group or a weakly acidic cation-exchange resin in which the cation-exchange group is a carboxy group, a phenolic hydroxy group, or the like.

However, a trace amount of acid can mix in the crude quaternary ammonium compound aqueous solution during its production process and the solution can exhibit acidity. Thus, a strongly acidic cation-exchange resin that can exchange ions also in a strongly acidic region is preferably used. Alternatively, the acid can be neutralized with a hydroxide having a quaternary ammonium ion identical to that of the crude quaternary ammonium compound aqueous solution, and then the solution is brought into contact with a weakly acidic cation-exchange resin.

The strongly acidic cation-exchange resin usually has a structure in which a sulfonic acid group is introduced into a cross-linked resin matrix composed of a copolymer of a cation-exchange group-introduced precursor monovinyl monomer and a cross-linking monomer. Examples of such a cation-exchange group-introduced precursor monovinyl monomer include styrene; an alkyl-substituted styrene, such as methylstyrene and ethylstyrene; and halogen-substituted styrene, such as bromostyrene. One of these can be used alone, or two or more can be mixed and used. The monovinyl monomer is, among these, particularly preferably styrene or a monomer mainly composed of styrene.

In addition, examples of the cross-linking monomer include a cross-linking monomer having a plurality of vinyl groups, such as divinylbenzene, trivinylbenzene, divinyltoluene, divinylnaphthalene, divinylxylene, divinylbiphenyl, bis(vinylphenyl)methane, bis(vinylphenyl)ethane, bis(vinylphenyl)propane, and bis(vinylphenyl)butane. One of these can be used alone, or two or more can be mixed and used. The cross-linking monomer is, among these, particularly preferably divinylbenzene.

On the other hand, examples of the weakly acidic cation-exchange resin include a copolymer of a monovinyl monomer having the weakly acidic cation-exchange group, such as acrylic acid or methacrylic acid, and a cross-linking monomer. The weakly acidic cation-exchange resin is particularly preferably an acrylic acid-divinylbenzene copolymer or a methacrylic acid-divinylbenzene copolymer.

The greatest feature of the present invention is using a cation-exchange resin with a degree of cross-linking of 6 or more in which a counter ion is a non-metal ion type as the cation-exchange resin with which the crude quaternary ammonium compound aqueous solution is brought into contact. That maintains the effect of removing the metal ion at a high level even though the crude quaternary ammonium compound aqueous solution is continued to be treated in a large amount. Here, the degree of cross-linking of the cation-exchange resin is determined by calculating [(weight of cross-linking monomer/weight of total monomers placed) x 100] in producing the resin. When the cation-exchange resin is a strongly acidic cation-exchange resin, it is often the case that a cross-linked resin matrix is first synthesized and a cation-exchange group is introduced by post-treatment as described above. In the strongly acidic cation-exchange resin, the degree of cross-linking is calculated without considering the weight of the cation-exchange group that is often introduced in the post-treatment and without including the weight in the above equation. When the degree of cross-linking of the cation-exchange resin to be used is unknown, the degree of cross-linking is calculated by determining the ratio between the cross-linking monomer and another monomer by subjecting the cation-exchange resin to pyrolysis gas chromatography-mass spectrometry (GC/MS).

Here, the action of maintaining the effect of removing the metal ion at a high level using the cation-exchange resin with a degree of cross-linking of 6 or more is an effect specifically exhibited when a crude quaternary ammonium compound aqueous solution with a low content of the organic impurity with a large molecular weight is used as the crude quaternary ammonium compound aqueous solution. In other words, in the treatment for a photoresist developer waste liquid as performed in Patent Document 2, the organic impurities with large molecular weights are contained in large amounts, and thus this effect is hardly exhibited.

The reason why the effect of continuously maintaining the effect of removing the metal ion is specifically exhibited only in the crude quaternary ammonium compound aqueous solution with a low content of the organic impurity with a large molecular weight is not necessarily clear, but the present inventors presume that this is due to the following reason. That is, when the cation-exchange resin becomes a highly cross-linked body, this increases the entanglement of the polymer chains constituting the resin and in turn increases the resin density. Thus, ion diffusion into the ion-exchange resin becomes difficult.

When the crude quaternary ammonium compound aqueous solution is brought into contact with the cation-exchange resin, for an ion with a minute ion size (ion radius of 2 Å or less), such as a hydrogen ion or a metal ion, among cations, the diffusibility into the interior of the particles does not greatly change even though the resin density increases. On the other hand, an ion with a large ion size, such as a quaternary ammonium ion, can easily diffuse into the interior of the particles if the resin density is low; however, the increasing degree of cross-linking and the increasing resin density gradually inhibit the internal diffusibility. This is presumed to result in preventing the internal diffusion of the quaternary ammonium ion in the highly cross-linked cation-exchange resin, thus exhibiting the adsorption selectivity for the metal ion with high diffusibility. Thus, using the cation-exchange resin with a degree of cross-linking of 6 or more adsorb the metal ion impurity with higher selectivity than the quaternary ammonium ion present in a large excess in the liquid to be treated and can reduce the metal ion concentration. The above mechanism becomes more significant particularly as the carbon number of the alkyl group of the quaternary ammonium ion increases. In addition, when the total of the carbon number of each alkyl group of the quaternary ammonium ion is the same, the above mechanism tends to become significant for the ion of (i), (ii), or (iii) above than for the ion in which all the alkyl groups are identical.

However, this selective and excellent removing action is significantly exhibited only at the initial stage of the purification treatment for the crude quaternary ammonium compound aqueous solution containing the organic impurity with a molecular weight of 1000 g/mol or more in a large amount more than 1000 ppm. This is considered to be caused because the long molecular chains of the organic impurity are homogeneously dispersed in the crude quaternary ammonium compound aqueous solution until the initial stage of the purification treatment and hardly affect the adsorption properties of the cation-exchange resin, but as the purification is continued, the long molecular chains gradually become entangled with the surface of the resin particles and eventually cover most of the surface. That is, it is presumed that when most of the particle surface of the cation-exchange resin is thus covered with the organic impurity, even the metal ion is strongly inhibited from diffusing into the interior of the cation-exchange resin, and the selective removing action is not smoothly exhibited.

From the viewpoint of more highly exhibiting the effect of removing the metal ion, the degree of cross-linking of the cation-exchange resin is preferably 8 or more, more preferably 11 or more, and particularly preferably 16 or more. In particular, when the ratio (liquid flow amount) of the crude quaternary ammonium compound aqueous solution to the cation-exchange resin described below exceeds 200 (L/L-resin), the degree of cross-linking of the cation-exchange resin is preferably 8 or more. On the other hand, when the content of the organic impurity with a molecular weight of 1000 g/mol or more in the crude quaternary ammonium compound aqueous solution is below 100 ppm, a sufficient effect is achieved even though the liquid flow amount exceeds 200 (L/L-resin) and the degree of cross-linking of the cation-exchange resin is 6 or more. However, too high a degree of cross-linking of the cation-exchange resin reduces the diffusion rate of even the metal ion and in turn reduces the capture rate of the metal. Thus, the degree of cross-linking is preferably 30 or less, more preferably 25 or less, and particularly preferably 22 or less.

The form of the cation-exchange resin like this can be any form, such as a gel type or a macroporous type. However, larger contact area between the ion-exchange resin and the liquid facilitates the diffusion of the metal ion into the ion-exchange resin, and thus a macroporous type is more suitably used in the present invention.

Additionally, the cation-exchange resin used in the present invention preferably has a cation exchange capacity of 1.0 to 5.0 equiv/L, and more preferably the cation-exchange resin with a cation exchange capacity in a range of 1.5 to 3.0 equiv/L is used. The form of the resin body of the cation-exchange resin is not particularly limited and can be a membrane form or the like but is usually preferably a particulate form.

Here, for the counter ion of the cation-exchange resin, a non-metal ion type needs to be used. That is, the counter ion of the cation-exchange resin is usually a hydrogen ion type or a metal ion type; however, a metal ion type among these cannot be used in the present invention because a metal ion present as the counter ion is eluted in the purification step and interferes with the reduction of the metal ion concentration in a purified quaternary ammonium compound aqueous solution to be produced. Thus, for the counter ion of the cation-exchange resin, a cation other than a metal ion, specifically, a cation composed of an ammonium ion (NH₄⁺), a primary to quaternary ammonium ion, and/or the like is used in addition to the hydrogen ion.

In the present invention, the counter ion of the cation-exchange resin is preferably a mixture type of a quaternary ammonium ion identical to that of the crude quaternary ammonium compound and a hydrogen ion type, or a hydrogen ion type, and more preferably a mixture type of a quaternary ammonium ion identical to that of the crude quaternary ammonium compound aqueous solution and a hydrogen ion type. In the case where the counter ion is an ammonium ion (NH₄⁺), a primary to quaternary ammonium ion type, and/or the like, a purified quaternary ammonium compound aqueous solution to be produced may be contaminated with a different type of ammonium compound due to elution of the different type of cation except for a state where the cation is a quaternary ammonium ion identical to that of the crude quaternary ammonium compound aqueous solution, and if this occurs, separation would become difficult.

When only a cation-exchange resin in which the counter ion is a metal ion type can be prepared, when a metal ion is contained in a certain amount and the metal ion concentration is desired to be reduced, or in other cases, the cation-exchange resin is brought into contact with an aqueous solution containing a cation other than the metal ion to remove the metal ion, and the cation-exchange resin with a degree of cross-linking of 6 or more in which a counter ion is a non-metal ion type can be prepared as a non-metal ion type, accordingly. However, as described above, in a cation-exchange resin with a high degree of cross-linking, a large-size cation, such as a quaternary ammonium ion, hardly diffuses into the resin interior. Thus, a measure of bringing the cation-exchange resin into contact with an aqueous solution containing a small-size cation that easily enters the resin interior and can be rapidly exchanged for the metal ion is taken into consideration. A preferred form of that is an aqueous solution in which the cation is a hydrogen ion, that is, a measure of bringing into contact with an acid is taken into consideration.

In addition, a particularly suitable aspect of the present invention uses a cation-exchange resin prepared by bringing such a cation-exchange resin with a degree of cross-linking of 6 or more in which a counter ion is a hydrogen ion type into contact with a quaternary ammonium compound aqueous solution having a quaternary ammonium ion identical to that of the crude quaternary ammonium compound aqueous solution. That is, according to this treatment, in the cation-exchange resin, some of the counter ions remain as hydrogen ions due to the influence by the diffusion inhibition of the quaternary ammonium ion, but a considerable amount of the hydrogen ions is exchanged for the quaternary ammonium ions identical to those of the crude quaternary ammonium compound aqueous solution. Thus, in a cation-exchange resin to be produced, a considerable proportion of the counter ions are exchanged for quaternary ammonium ions identical to those of the crude quaternary ammonium compound aqueous solution. The cation-exchange resin in such a state can prevent the phenomenon in which the quaternary ammonium ion of the crude quaternary ammonium compound aqueous solution to be treated is adsorbed to the cation-exchange resin at the start of purification of the crude quaternary ammonium compound aqueous solution according to the present invention and a purified quaternary ammonium compound aqueous solution is produced temporarily with lower concentration. This prevention is suitable.

Also after the cation-exchange resin is brought into contact with the quaternary ammonium compound aqueous solution having a quaternary ammonium ion identical to that of the crude quaternary ammonium compound aqueous solution as described above, some of the hydrogen ions remain as the counter ions of the highly cross-linked cation-exchange resin. For a suitable residual amount of the hydrogen ion, preferably at least 1 mol% is a hydrogen ion type, more preferably at least 3 mol%, even more preferably at least 10 mol%, and particularly preferably at least 20 mol% is a hydrogen ion type. With a larger amount of the residual hydrogen ion, the amount of the crude quaternary ammonium compound aqueous solution to be treated can be increased, which is thus preferred. In addition, the proportion in which the counter ion of the cation-exchange resin is a quaternary ammonium ion identical to that of the crude quaternary ammonium compound is preferably 99 mol% or less of the counter ion, more preferably 97 mol% or less, even more preferably 90 mol% or less, particularly preferably 80 mol% or less, and the rest of the counter ion is a hydrogen ion type.

On the other hand, bringing the crude quaternary ammonium compound aqueous solution into contact with a hydrogen ion type cation-exchange resin also allows purification without eluting the metal. However, if the crude quaternary ammonium compound aqueous solution is purified with the cation-exchange resin in such a state, the quaternary ammonium ions of the liquid to be treated are adsorbed to the cation-exchange resin, and the concentration of a purified quaternary ammonium compound aqueous solution temporarily decreases. In addition, the same amount of hydrogen ions as the adsorption amount is eluted, and thus the acid concentration temporarily increases, causing contamination. Thus, it is suitable to bring the cation-exchange resin into contact with a quaternary ammonium compound aqueous solution having a quaternary ammonium ion identical to that of the crude quaternary ammonium compound aqueous solution before purification to exchange a counter ion for the quaternary ammonium ion in advance, the counter ion other than a hydrogen ion remaining due to the diffusion inhibition. The proportion of quaternary ammonium ions to exchange groups in the cation-exchange resin before purification, the exchange groups exchangeable for quaternary ammonium ions identical to those of the crude quaternary ammonium compound aqueous solution, is preferably 60 mol% or more, more preferably 80 mol% or more, even more preferably 95 mol% or more, and particularly preferably 99 mol% or more.

As described above, the counter ion of the cation-exchange resin is a non-metal ion type, but this does not mean that all the counter ions are completely other than a metal ion, and it is allowed that a metal ion is contained to the extent that it inevitably mixes in as long as the effect is not greatly affected. Specifically, if the content of a metal ion is about 3 mol% or less, suitably about 0.1 mol% or less, and particularly suitably about 0.001 mol% or less of the counter ion, this is allowed as the scope of the present invention.

Here, in the present invention, for the counter ion of the cation-exchange resin, the cation-exchange resin is brought into contact with an acid to elute a metal ion and a non-metal ion other than a hydrogen ion, and each component can be quantified with an ion chromatograph. In addition, a hydrogen ion can be quantified by bringing the cation-exchange resin into contact with a salt to elute a hydrogen ion and performing neutralization titration.

Here, for the acid aqueous solution used for contact with the cation-exchange resin in which the counter ion is a metal ion type, an inorganic acid aqueous solution of sulfuric acid, hydrochloric acid, nitric acid, or the like is used, and from the viewpoint of preventing deterioration of the resin material, a hydrochloric acid aqueous solution or a sulfuric acid aqueous solution is preferably used. In addition, the acid aqueous solution can be used without limitation on the concentration of the acid. However, contact with a high concentration acid replaces a counter ion in a short time and may cause cracking or breakage due to a rapid volumetric change. Thus, the concentration is preferably 1 mol/L or less, more preferably 0.5 mol/L or less, and even more preferably 0.3 mol/L or less. Furthermore, from the viewpoint of sufficiently eluting ions other than a hydrogen ion contained in the counter ions of the cation-exchange resin, the cation-exchange resin is preferably treated with an acid equivalent to 1.2 to 5 times and more preferably 1.5 to 3.5 times the total amount of exchange groups of the cation-exchange resin to be used.

The contact treatment is performed by a batch method or a liquid flow method without particular limitation. In the case of a liquid flow method in which a resin column is formed, the liquid flow can be either upward flow or downward flow, and an acid is preferably passed through in an amount equivalent to three or more times the amount of exchange groups of the ion-exchange resin to be treated. Although the liquid flow rate is not particularly specified, the liquid flow is preferably performed in a range of SV = 1 to 20 (1/hr) in terms of treatment time and efficiency. In addition, in the case of a batch method, the liquid is exchanged three times or more with an acid equivalent to 3 to 15 times the total amount of exchange groups of the cation-exchange resin to be treated.

Furthermore, if a metal ion is contained in a large amount in the acid aqueous solution, the metal ion remains in the cation-exchange resin depending on the concentration of the metal ion. This may reduce the exchange amount of the metal ion during purification or release the metal ion and cause contamination in some cases. Thus, the total content of the metal impurity composed of Na, K, Li, Ca, Mg, and Sr contained in the acid aqueous solution is preferably 100 ppt or less, more preferably 10 ppt or less, and even more preferably 1 ppt or less. Moreover, in the acid aqueous solution, the total content of a metal impurity composed of Fe, Ni, Cu, and Pb is also preferably 100 ppt or less, more preferably 10 ppt or less, and even more preferably 1 ppt or less.

After contact with the acid aqueous solution, the cation-exchange resin is preferably washed with ultrapure water. This removes an unnecessary acid that has not entered as a counter ion due to adhesion to the surface of the resin or the like and can prevent the acid from mixing in a later step. In particular, in the case where the quaternary ammonium compound contained in the crude quaternary ammonium compound aqueous solution is a quaternary ammonium hydroxide, if an acid remains in the cation-exchange resin, rapid heat generation due to a neutralization reaction may occur, and resin breakage due to rapid swelling of the resin may occur. Thus, the cation-exchange resin is preferably washed with ultrapure water.

In addition, for the quaternary ammonium compound aqueous solution used when the cation-exchange resin in which a counter ion is a hydrogen ion type is brought into contact with a quaternary ammonium compound aqueous solution having a quaternary ammonium ion identical to that of the crude quaternary ammonium compound aqueous solution, the same concentration, contact method, and metal ion content as the requirements described for the acid aqueous solution are suitably used. In particular, treating the cation-exchange resin with a quaternary ammonium compound aqueous solution having a quaternary ammonium ion identical to that of the crude quaternary ammonium compound aqueous solution, the quaternary ammonium compound aqueous solution in an amount equivalent to 1.5 to 3.5 times the total amount of exchange groups of the cation-exchange resin to be used, makes the proportion of the quaternary ammonium ions to the exchange groups in the cation-exchange resin, the exchange groups exchangeable for quaternary ammonium ions identical to those of the crude quaternary ammonium compound aqueous solution, 99 mol% or more, which is preferred. In addition, for the quaternary ammonium compound aqueous solution having a quaternary ammonium ion identical to that of the crude quaternary ammonium compound aqueous solution, a hydroxide, a chloride, a bromide salt, a carbonate, or the like can be used, but a hydroxide is preferred. In the case of a hydroxide, a hydrogen ion eluted from the cation-exchange resin immediately forms a hydroxide ion and water. Thus, a hydrogen ion is efficiently exchanged for a quaternary ammonium ion without increasing the hydrogen ion concentration in the liquid. Furthermore, as in the case after contact with the acid aqueous solution, the cation-exchange resin is preferably washed with ultrapure water also after contact with the quaternary ammonium compound aqueous solution.

Next, in the present invention, the method of bringing the cation-exchange resin into contact with the crude quaternary ammonium compound aqueous solution can be in any form, such as a batch method or a liquid flow method, but is preferably a liquid flow method from the industrial viewpoint. In the liquid flow method, purification is performed by providing a resin column and passing the solution. The liquid flow direction can be either upward flow or downward flow and is preferably downward flow from the viewpoint of increasing purification efficiency. The liquid flow condition is preferably a condition of a space velocity SV = 1 to 20 (1/hr), more preferably a condition of 5 to 20 (1/hr), and even more preferably a condition of 8 to 20.

For the treatment amount of the crude quaternary ammonium compound aqueous solution to be brought into contact, usually 100 (L/L-resin) or more is efficient. As described above, the excellent effect of removing the metal ion in the method of the present invention is significantly exhibited when the treatment amount of the crude quaternary ammonium compound aqueous solution amounts to a large quantity. Thus, the treatment amount achieving a condition where the crude quaternary ammonium compound aqueous solution to the cation-exchange resin is 2000 (L/L-resin) or more and more preferably 10000 (L/L-resin) or more is more effective. However, if the treatment amount increases too much, the effect of removing the metal ion is reduced. Thus, the treatment amount is usually 10⁸ (L/L-resin) or less and particularly preferably 50000 (L/L-resin) or less.

For the cation-exchange resin whose effect of removing the metal ion has been thus reduced, it is efficient to subject such a cation-exchange resin to the acid treatment as a resin regeneration treatment, then resume the liquid flow, and repeat purification. In addition, the solution can be passed through two or more resin columns in series.

On the other hand, in the purification by a batch method, the crude quaternary ammonium compound aqueous solution is preferably immersed in an amount of 200 L/L-resin or more from the viewpoint of significantly exhibiting the excellent effect of removing the metal ion. In addition, repeating the batch treatment can further decrease the metal ion, which is thus preferred. The number of repetitions is favorably two to five times.

In the method of the present invention, when a particulate metal impurity is contained in the crude quaternary ammonium compound aqueous solution, the solution is preferably subjected to filter treatment as a step before or after the contact with the cation-exchange resin to remove the impurity. If a particulate metal impurity or a resin particle is contained in the crude quaternary ammonium compound aqueous solution, this may interfere with the action in the removal of the metal ion by the cation-exchange resin in the present invention or may cause clogging in the case of a liquid flow method. Thus, the solution is preferably subjected to filter treatment as a pretreatment. In addition, in the filter treatment, a particulate metal impurity remaining after contact with the cation-exchange resin and, in some cases, a broken piece of the cation-exchange resin may mix in. Thus, the filter treatment is also preferably performed as a post-treatment.

The average pore diameter of the filter to be used is preferably 1 µm or less and more preferably from 0.02 to 1 µm. When the average pore diameter of the filter is unknown, a value measured by a bubble point method is used as the average pore diameter.

The inner wall of an apparatus, such as a container, a tank, a resin column, or a pipe used for carrying out the production method of the present invention is preferably made of a material having chemical resistance and preventing metal contamination as much as possible. Thus, the inner wall is preferably made of or lined with a material of polyethylene, polypropylene, or a fluororesin. In particular, a fluororesin is most suitable from the viewpoint of preventing metal contamination. The surfaces of these members are preferably washed in advance before use; they are preferably cleaned, for example, by washing with a quaternary ammonium compound hydroxide aqueous solution and/or an acid.

The method described above effectively produces a purified quaternary ammonium compound aqueous solution in which the metal ion is decreased. Specifically, it is also possible to prepare a quaternary ammonium compound aqueous solution with a total content of metal impurities composed of Na, K, Li, Ca, Mg, and Sr of 100 ppt or less, more preferably 50 ppt or less, and even more preferably 20 ppt or less. The content of each of these metal elements in the quaternary ammonium compound aqueous solution can also be 30 ppt or less, more preferably 10 ppt or less, and even more preferably 5 ppt or less.

In addition, when the quaternary ammonium compound of the quaternary ammonium compound aqueous solution is a hydroxide salt, the quaternary ammonium compound aqueous solution is strongly alkaline. Thus, some metal elements other than the metal elements described above may be in the form of an anion or a non-ion, and the effect of removing these metal elements may be insufficient. However, when the quaternary ammonium compound is a quaternary ammonium halide compound or a quaternary ammonium carbonate compound other than a hydroxide, the effect of decreasing each metal element other than the above is also highly exhibited. Thus, when the counter ion is other than a hydroxide, it is also possible to prepare a quaternary ammonium compound aqueous solution with a total content of metal impurities composed of Fe, Ni, Cu, and Pb of 100 ppt or less, more preferably 50 ppt or less, and even more preferably 20 ppt or less.

### Examples

Hereinafter, to specifically describe the present invention, examples will be shown, but the present invention is not limited to these examples. Properties in examples and comparative examples were measured by the following methods.

### 1) Content of Metal Impurities Contained in Quaternary Ammonium Compound Aqueous Solution

The concentrations of Na, K, Li, Ca, Mg, Sr, Fe, Ni, Cu, and Pb were measured with a high-frequency inductively coupled plasma mass spectrometer (ICP-MS). The lower limit of measurement was 1 ppt, and a measurement of less than 1 ppt was indicated as < 1 ppt.

### 2) Content of Organic Impurity with Molecular Weight of 1000 g/mol or More Contained in Quaternary Ammonium Compound Aqueous Solution

A sample of a crude quaternary ammonium compound aqueous solution was injected into a GPC column, and a liquid containing 1000 g/mol or more of an organic impurity was separated and recovered. Two liquids, the recovered liquid and an eluent used for the separation by GPC, were each measured with a TOC meter to determine TOC concentration. The TOC concentration of the eluent is subtracted from the TOC concentration of the recovered liquid, the resulting difference is multiplied by the dilution factor of the crude quaternary ammonium compound aqueous solution in the recovered liquid, thereby the concentration of an organic impurity in the crude quaternary ammonium compound aqueous solution is determined.

### 3) Proportions of Quaternary Ammonium Ions and Hydrogen Ions Contained in Counter Ions of cation-exchange resin

A cation-exchange resin subjected to a pretreatment shown in examples was brought into contact with sodium chloride to elute hydrogen ions, and the resulting liquid was subjected to neutralization titration with sodium hydroxide to quantify hydrogen ions contained in the counter ions. In addition, the same cation-exchange resin was brought into contact with hydrochloric acid (all counter ions were assumed to be hydrogen ions), then washed with ultrapure water, and brought into contact with sodium chloride again. The resulting liquid was subjected to neutralization titration with sodium hydroxide to quantify the hydrogen ions to determine the amount of exchange groups of the cation-exchange resin. The counter ions of the pretreated cation-exchange resin are composed of a quaternary ammonium ion and a hydrogen ion, and thus the amount of quaternary ammonium ions is determined by subtracting the amount of hydrogen ions initially determined from the amount of exchange groups. The amount of quaternary ammonium ions and the amount of hydrogen ions were each divided by the amount of exchange groups to determine the proportions.

### [Quaternary Ammonium Compound Aqueous Solution]

· Ethyltrimethylammonium chloride (prepared from raw materials)
· Ethyltrimethylammonium hydroxide (prepared from the ethyltrimethylammonium chloride)
· Tetramethylammonium chloride (commercially available product from Company A, common grade)
· Tetramethylammonium hydroxide (available from Tokuyama Corporation, 25% aqueous solution)

Ethyltrimethylammonium chloride was prepared by the following procedure. Trimethylamine and ethyl chloride were placed in a molar ratio of 1:1 in a reaction vessel containing ultrapure water and held at a reaction temperature of 60°C, and a 60 mass% ethyltrimethylammonium chloride aqueous solution was prepared.

Ethyltrimethylammonium hydroxide was prepared by the following procedure using an electrolytic cell having a power source 3 illustrated in FIG. 1. Electrolysis was carried out continuously using a platinum-plated nickel plate for a cathode 2, a platinum-plated titanium plate for an anode 1, two pieces of Nafion N324 (available from Chemours) for cation-exchange membranes 9, and ASE (available from Astom) for an anion-exchange membrane 8, and by circulating 0.5 N HCl in an anode chamber 4, an ethyltrimethylammonium chloride aqueous solution adjusted to 50 mass% in a raw material chamber 5 between the anion-exchange membrane 8 and a cation-exchange membrane 9 on the cathode side, and ultrapure water each in a cathode chamber 7 and an intermediate chamber 6 between the two cation-exchange membranes 9, while the current density was gradually increased and finally the current density was maintained at 30 A/dm² and the temperature was maintained at 40°C. During the electrolysis, the ethyltrimethylammonium chloride aqueous solution was supplemented to maintain the ethyltrimethylammonium chloride concentration in the raw material chamber 5 at 40 mass% or more, ultrapure water was added to adjust the concentration of ethyltrimethylammonium hydroxide produced in the cathode chamber 7 to be constant at 25 mass%, and the liquid was appropriately extracted from the cathode chamber 7, and ethyltrimethylammonium hydroxide was continuously prepared, accordingly. In addition, ethyltrimethylammonium hydroxide to be used for pretreatment of the cation-exchange resin was treated with a 0.3 mol/L hydrochloric acid and treated with ultrapure water, and then passed through Amberlite 200CT Na (degree of cross-linking 20, cation exchange capacity 1.7 equiv/L, available from Organo Corporation) to reduce the metal concentration.

### Example 1

For the prepared 60 mass% crude ethyltrimethylammonium chloride aqueous solution, the contents of metal impurities were measured. The total content of metal impurities composed of Na, K, Li, Ca, Mg, and Sr is shown in Table 1, and the total content of metal impurities composed of Fe, Ni, Cu, and Pb is shown in Table 2. In addition, the content of an organic impurity with a molecular weight of 1000 g/mol or more was measured and found to be 233 ppm.

Then, 50 mL of a strongly acidic ion-exchange resin, Amberlite 200CT Na (degree of cross-linking 20, cation exchange capacity 1.7 equiv/L, available from Organo Corporation), was packed in a column with a diameter of 22 mm x 750 mm. As a pretreatment, liquids were passed through this packed column in the following order:
1) ultrapure water washing,
2) 0.3 mol/L hydrochloric acid treatment,
3) ultrapure water treatment,
4) 0.3 mol/L ethyltrimethylammonium hydroxide aqueous solution treatment, and
5) ultrapure water treatment. The liquid flow amount of each liquid was 600 mL, and the space velocity was adjusted to SV = 5 (1/hr).

Each of the 0.3 mol/L hydrochloric acid and the 0.3 mol/L ethyltrimethylammonium hydroxide aqueous solution had properties of a total content of the metal impurities composed of Na, K, Li, Ca, Mg, and Sr of 100 ppt or less and a total content of the metal impurities composed of Fe, Ni, Cu, and Pb of also 100 ppt or less. In addition, for the ultrapure water, a liquid with a total content of the metal impurities composed of Na, K, Li, Ca, Mg, Sr, Fe, Ni, Cu, and Pb of 1 ppt or less was used. Furthermore, the proportions of quaternary ammonium ions and hydrogen ions contained in the counter ions of the strongly acidic ion-exchange resin after the pretreatment were measured to find that the proportion of quaternary ammonium ions was 77 mol% and the proportion of hydrogen ions was 23 mol%.

Through the column, 1050 L of a crude tetramethylammonium chloride aqueous solution was passed under a condition of SV = 20 (1/hr). Each time after the liquid flow amount reached 10 L (200 L/L-resin), 100 L (2000 L/L-resin), 500 L (10000 L/L-resin), and 1000 L (20000 L/L-resin), 10 L of the corresponding liquid was collected, and the contents of the metal impurities were measured. The results are each shown in Tables 1 and 2.

### Example 2

The same operation as in Example 1 was performed except for changing the strongly acidic ion-exchange resin to be used to Diaion UBK16 (degree of cross-linking 16, cation exchange capacity 2.3 equiv/L, Mitsubishi Chemical Corporation) in Example 1. The proportion of quaternary ammonium ions contained in the counter ions of the strongly acidic ion-exchange resin after the pretreatment at this time was 87 mol%, and the proportion of hydrogen ions was 13 mol%. The measurement results of the metal impurity contents are each shown in Tables 1 and 2 above.

### Example 3

The same operation as in Example 1 was performed except for changing the strongly acidic ion-exchange resin to be used to Diaion SK112 (degree of cross-linking 12, cation exchange capacity 2.1 equiv/L, available from Mitsubishi Chemical Corporation) in Example 1. The proportion of quaternary ammonium ions contained in the counter ions of the strongly acidic ion-exchange resin after the pretreatment at this time was 95 mol%, and the proportion of hydrogen ions was 5 mol%. The measurement results of the metal impurity contents are each shown in Tables 1 and 2 above.

### Example 4

The same operation as in Example 1 was performed except for changing the strongly acidic ion-exchange resin to be used to Diaion SK1B (degree of cross-linking 8, cation exchange capacity 2.0 equiv/L, available from Mitsubishi Chemical Corporation) in Example 1. The proportion of quaternary ammonium ions contained in the counter ions of the strongly acidic ion-exchange resin after the pretreatment at this time was 97 mol%, and the proportion of hydrogen ions was 3 mol%. The measurement results of the metal impurity contents are each shown in Tables 1 and 2 above.

### Example 5

The same operation as in Example 1 was performed except for changing the strongly acidic ion-exchange resin to be used to Diaion PK212 (degree of cross-linking 6, cation exchange capacity 1.5 equiv/L, available from Mitsubishi Chemical Corporation) in Example 1. The proportion of quaternary ammonium ions contained in the counter ions of the strongly acidic ion-exchange resin after the pretreatment at this time was 99 mol%, and the proportion of hydrogen ions was 1 mol%. The measurement results of the metal impurity contents are each shown in Tables 1 and 2 above.

**[Table 1-1]**

| | | Metal (ppt) | | | | | | |
|---|---|---|---|---|---|---|---|---|
| | | Na | K | Li | Ca | Mg | Sr | Total |
| 60% Ethyltrimethylammonium chloride aqueous solution (before purification) [content of large-molecular-weight organic impurity* 233 ppm] | | 4.8 x 10⁴ | 3.5 x 10³ | 1.1 x 10³ | 3.7 x 10⁴ | 8.8 x 10² | 1.0 x 10² | 9.1 x 10⁴ |
| Example 1 | 200 L/L-resin | < 1 | < 1 | < 1 | < 1 | < 1 | < 1 | 6 |
| | 2000 L/L-resin | < 1 | < 1 | < 1 | < 1 | < 1 | < 1 | 6 |
| | 10000 L/L-resin | 2 | < 1 | < 1 | < 1 | < 1 | < 1 | 7 |
| | 20000 L/L-resin | 3 | < 1 | < 1 | < 1 | < 1 | < 1 | 8 |
| Example 2 | 200 L/L-resin | < 1 | < 1 | < 1 | < 1 | < 1 | < 1 | 6 |
| | 2000 L/L-resin | < 1 | < 1 | < 1 | < 1 | < 1 | < 1 | 6 |
| | 10000 L/L-resin | 2 | 2 | < 1 | < 1 | < 1 | < 1 | 8 |
| | 20000 L/L-resin | 4 | 3 | < 1 | < 1 | < 1 | < 1 | 11 |
| Example 3 | 200 L/L-resin | 2 | < 1 | < 1 | < 1 | < 1 | < 1 | 7 |
| | 2000 L/L-resin | 4 | 2 | < 1 | < 1 | < 1 | < 1 | 10 |
| | 10000 L/L-resin | 8 | 7 | 2 | < 1 | < 1 | < 1 | 20 |
| | 20000 L/L-resin | 23 | 20 | 7 | 3 | < 1 | < 1 | 55 |
| Example 4 | 200 L/L-resin | 3 | < 1 | < 1 | < 1 | < 1 | < 1 | 8 |
| | 2000 L/L-resin | 7 | 2 | < 1 | < 1 | < 1 | < 1 | 13 |
| | 10000 L/L-resin | 22 | 15 | 6 | 3 | < 1 | < 1 | 48 |
| | 20000 L/L-resin | 4.8 x 10⁴ | 3.5 x 10³ | 1.1 x 10³ | 3.7 x 10⁴ | 8.8 x 10² | 1.0 x 10² | 9.1 x 10⁴ |
| Example 5 | 200 L/L-resin | 43 | 19 | 11 | 4 | < 1 | < 1 | 79 |
| | 2000 L/L-resin | 4.8 x 10⁴ | 3.5 x 10³ | 1.1 x 10³ | 3.7 x 10⁴ | 8.8 x 10² | 1.0 x 10² | 9.1 x 10⁴ |
| | 10000 L/L-resin | 4.8 x 10⁴ | 3.5 x 10³ | 1.1 x 10³ | 3.7 x 10⁴ | 8.8 x 10² | 1.0 x 10² | 9.1 x 10⁴ |
| | 20000 L/L-resin | 4.8 x 10⁴ | 3.5 x 10³ | 1.1 x 10³ | 3.7 x 10⁴ | 8.8 x 10² | 1.0 x 10² | 9.1 x 10⁴ |

| | | | | | | | | |
|---|---|---|---|---|---|---|---|---|
| * Large-molecular-weight organic impurity: organic impurity with a molecular weight of 1000 g/mol or more | | | | | | | | |

**[Table 2]**

| | | Metal (ppt) | | | | |
|---|---|---|---|---|---|---|
| | | Fe | Ni | Cu | Pb | Total |
| 60% Ethyltrimethylammonium chloride aqueous solution (before purification) [content of large-molecular-weight organic impurity* 233 ppm] | | 2.2 x 10³ | 1.7 x 10⁴ | 6.3 x 10² | 7.3 x 10 | 2.0 x 10⁴ |
| Example 1 | 200 L/L-resin | < 1 | < 1 | < 1 | < 1 | 4 |
| | 2000 L/L-resin | 2 | < 1 | < 1 | < 1 | 5 |
| | 10000 L/L-resin | 2 | < 1 | 2 | < 1 | 6 |
| | 20000 L/L-resin | 3 | < 1 | 3 | < 1 | 8 |
| Example 2 | 200 L/L-resin | 2 | < 1 | < 1 | < 1 | 5 |
| | 2000 L/L-resin | 2 | < 1 | < 1 | < 1 | 5 |
| | 10000 L/L-resin | 3 | < 1 | 2 | < 1 | 7 |
| | 20000 L/L-resin | 4 | 2 | 4 | < 1 | 11 |
| Example 3 | 200 L/L-resin | 2 | < 1 | 2 | < 1 | 6 |
| | 2000 L/L-resin | 3 | < 1 | 3 | < 1 | 8 |
| | 10000 L/L-resin | 5 | < 1 | 5 | < 1 | 12 |
| | 20000 L/L-resin | 17 | 7 | 19 | < 1 | 44 |
| Example 4 | 200 L/L-resin | 3 | < 1 | 2 | < 1 | 7 |
| | 2000 L/L-resin | 5 | < 1 | 5 | < 1 | 11 |
| | 10000 L/L-resin | 21 | 6 | 17 | < 1 | 45 |
| | 20000 L/L-resin | 2.2 x 10³ | 1.7 x 10⁴ | 6.3 x 10² | 7.3 x 10 | 2.0 x 10⁴ |
| Example 5 | 200 L/L-resin | 31 | 21 | 19 | < 1 | 72 |
| | 2000 L/L-resin | 2.2 x 10³ | 1.7 x 10⁴ | 6.3 x 10² | 7.3 x 10 | 2.0 x 10⁴ |
| | 10000 L/L-resin | 2.2 x 10³ | 1.7 x 10⁴ | 6.3 x 10² | 7.3 x 10 | 2.0 x 10⁴ |
| | 20000 L/L-resin | 2.2 x 10³ | 1.7 x 10⁴ | 6.3 x 10² | 7.3 x 10 | 2.0 x 10⁴ |

### Comparative Example 1

A 60 mass% crude ethyltrimethylammonium chloride aqueous solution was prepared using ethyltrimethylammonium chloride prepared separately from that of Example 1. The total content of metal impurities composed of Na, K, Li, Ca, Mg, and Sr of the crude ethyltrimethylammonium chloride aqueous solution is shown in Table 3, and the total content of metal impurities composed of Fe, Ni, Cu, and Pb is shown in Table 4. In addition, the content of an organic impurity with a molecular weight of 1000 g/mol or more was measured and found to be 1721 ppm.

For the crude tetramethylammonium chloride aqueous solution thus prepared, the metal impurity contents were measured by the same operation as in Example 1. The liquids for measuring the metal impurity contents were collected at 10 L (200 L/L-resin) and 100 L (2000 L/L-resin). The measurement results of the metal impurity contents are each shown in Tables 3 and 4.

**[Table 3]**

| | | Metal (ppt) | | | | | | |
|---|---|---|---|---|---|---|---|---|
| | | Na | K | Li | Ca | Mg | Sr | Total |
| 60% Ethyltrimethylammonium chloride aqueous solution (before purification) [content of large-molecular-weight organic impurity* 1721 ppm] | | 5.1 x 10⁴ | 1.9 x 10⁴ | 1.2 x 10³ | 7.2 x 10⁴ | 8.0 x 10³ | 1.1 x 10² | 1.5 x 10⁵ |
| Comparative Example 1 | 200 L/L-resin | 79 | 61 | 14 | 47 | 8 | < 1 | 210 |
| | 2000 L/L-resin | 5.1 x 10⁴ | 1.9 x 10⁴ | 1.2 x 10³ | 7.2 x 10⁴ | 8.0 x 10³ | 1.1 x 10² | 1.5 x 10⁵ |

**[Table 4]**

| | | Metal (ppt) | | | | |
|---|---|---|---|---|---|---|
| | | Fe | Ni | Cu | Pb | Total |
| 60% Ethyltrimethylammonium chloride aqueous solution (before purification) [content of large-molecular-weight organic impurity* 1721 ppm] | | 3.6 x 10³ | 2.4 x 10⁴ | 1.9 x 10³ | 1.4 x 10² | 3.0 x 10⁴ |
| Comparative Example 1 | 200 L/L-resin | 15 | 11 | 8 | < 1 | 35 |
| | 2000 L/L-resin | 3.6 x 10³ | 2.4 x 10⁴ | 1.9 x 10³ | 1.4 x 10² | 3.0 x 10⁴ |

### Examples 6 to 10

For prepared 25 mass% crude ethyltrimethylammonium hydroxide aqueous solutions, the metal impurity contents were measured. The results are each shown in Tables 5 and 6. In addition, the content of an organic impurity with a molecular weight of 1000 g/mol or more was also measured and found to be 26 ppm.

The same operations as in Examples 1 to 5 were performed except for using the crude tetramethylammonium hydroxide aqueous solutions thus prepared (Example 6 corresponds to Example 1, Example 7 corresponds to Example 2, Example 8 corresponds to Example 3, Example 9 corresponds to Example 4, and Example 10 corresponds to Example 5). Each measurement result of the metal impurity contents is shown each in Tables 5 and 6.

**[Table 5]**

| | | Metal (ppt) | | | | | | |
|---|---|---|---|---|---|---|---|---|
| | | Na | K | Li | Ca | Mg | Sr | Total |
| 25% Ethyltrimethylammonium hydroxide aqueous solution (before purification) [content of large-molecular-weight organic impurity 26 ppm] | | 103 | 81 | 8 | 179 | 5 | 3 | 378 |
| Example 6 | 200 L/L-resin | < 1 | < 1 | < 1 | 1 | < 1 | < 1 | 6 |
| | 2000 L/L-resin | < 1 | < 1 | < 1 | 1 | < 1 | < 1 | 6 |
| | 10000 L/L-resin | 1 | 1 | < 1 | 2 | < 1 | < 1 | 7 |
| | 20000 L/L-resin | 2 | 2 | < 1 | 3 | < 1 | < 1 | 10 |
| Example 7 | 200 L/L-resin | < 1 | < 1 | < 1 | 1 | < 1 | < 1 | 6 |
| | 2000 L/L-resin | 1 | 1 | < 1 | 1 | < 1 | < 1 | 6 |
| | 10000 L/L-resin | 2 | 2 | < 1 | 2 | < 1 | < 1 | 9 |
| | 20000 L/L-resin | 4 | 3 | < 1 | 3 | < 1 | < 1 | 13 |
| Example 8 | 200 L/L-resin | 1 | 1 | < 1 | 1 | < 1 | < 1 | 6 |
| | 2000 L/L-resin | 2 | 1 | < 1 | 2 | < 1 | < 1 | 8 |
| | 10000 L/L-resin | 3 | 2 | < 1 | 3 | < 1 | < 1 | 11 |
| | 20000 L/L-resin | 6 | 4 | < 1 | 5 | < 1 | < 1 | 18 |
| Example 9 | 200 L/L-resin | 1 | 1 | < 1 | 2 | < 1 | < 1 | 7 |
| | 2000 L/L-resin | 2 | 2 | < 1 | 2 | < 1 | < 1 | 9 |
| | 10000 L/L-resin | 4 | 3 | 1 | 4 | < 1 | < 1 | 14 |
| | 20000 L/L-resin | 7 | 5 | 1 | 7 | 1 | < 1 | 22 |
| Example 10 | 200 L/L-resin | 3 | 2 | < 1 | 4 | < 1 | < 1 | 12 |
| | 2000 L/L-resin | 12 | 9 | 1 | 16 | 1 | 1 | 40 |
| | 10000 L/L-resin | 101 | 80 | 3 | 172 | 5 | 3 | 368 |
| | 20000 L/L-resin | 103 | 81 | 8 | 179 | 5 | 3 | 378 |

**[Table 6]**

| | | Metal (ppt) | | | | |
|---|---|---|---|---|---|---|
| | | Fe | Ni | Cu | Pb | Total |
| 25% Ethyltrimethylammonium hydroxide aqueous solution (before purification) [content of large-molecular-weight organic impurity 26 ppm] | | 35 | 43 | 7 | 4 | 89 |
| Example 6 | 200 L/L-resin | 4 | 4 | 2 | 2 | 12 |
| | 2000 L/L-resin | 4 | 5 | 2 | 2 | 13 |
| | 10000 L/L-resin | 4 | 5 | 2 | 2 | 13 |
| | 20000 L/L-resin | 5 | 6 | 2 | 2 | 15 |
| Example 7 | 200 L/L-resin | 4 | 4 | 2 | 2 | 12 |
| | 2000 L/L-resin | 4 | 5 | 2 | 2 | 13 |
| | 10000 L/L-resin | 5 | 6 | 3 | 2 | 16 |
| | 20000 L/L-resin | 5 | 6 | 3 | 2 | 16 |
| Example 8 | 200 L/L-resin | 4 | 4 | 2 | 2 | 12 |
| | 2000 L/L-resin | 5 | 5 | 2 | 2 | 14 |
| | 10000 L/L-resin | 6 | 6 | 3 | 3 | 18 |
| | 20000 L/L-resin | 6 | 7 | 3 | 3 | 19 |
| Example 9 | 200 L/L-resin | 4 | 5 | 2 | 3 | 14 |
| | 2000 L/L-resin | 6 | 7 | 3 | 3 | 19 |
| | 10000 L/L-resin | 7 | 8 | 3 | 3 | 21 |
| | 20000 L/L-resin | 9 | 10 | 3 | 3 | 25 |
| Example 10 | 200 L/L-resin | 5 | 8 | 3 | 3 | 19 |
| | 2000 L/L-resin | 7 | 11 | 4 | 3 | 25 |
| | 10000 L/L-resin | 33 | 41 | 7 | 4 | 85 |
| | 20000 L/L-resin | 35 | 43 | 7 | 4 | 89 |

### Comparative Example 2

A powder of synthetic tetramethylammonium chloride (commercially available product from Company A, common grade) was dissolved with water and adjusted to 60% by mass, and a crude tetramethylammonium chloride aqueous solution was prepared. For this crude tetramethylammonium chloride aqueous solution, the contents of metal impurities were measured. The total content of metal impurities composed of Na, K, Li, Ca, Mg, and Sr is shown in Table 7, and the total content of metal impurities composed of Fe, Ni, Cu, and Pb is shown in Table 8. In addition, the content of an organic impurity with a molecular weight of 1000 g/mol or more was also measured and found to be 372 ppm.

Then, 50 mL of a strongly acidic ion-exchange resin, Amberlite 200CT Na (degree of cross-linking 20, cation exchange capacity 1.7 equiv/L, available from Organo Corporation), was packed in a column with a diameter of 22 mm x 750 mm. As a pretreatment, liquids were passed through this packed column in the following order:
1) ultrapure water washing,
2) 0.3 mol/L hydrochloric acid treatment,
3) ultrapure water treatment,
4) 0.3 mol/L tetramethylammonium hydroxide aqueous solution treatment, and
5) ultrapure water treatment. The liquid flow amount of each liquid was 600 mL, and the space velocity was adjusted to SV = 5 (1/hr).

Each of the 0.3 mol/L hydrochloric acid and the 0.3 mol/L tetramethylammonium hydroxide aqueous solution had properties of a total content of the metal impurities composed of Na, K, Li, Ca, Mg, and Sr of 100 ppt or less and a total content of the metal impurities composed of Fe, Ni, Cu, and Pb of also 100 ppt or less. In addition, for the ultrapure water, a liquid with a total content of the metal impurities composed of Na, K, Li, Ca, Mg, Sr, Fe, Ni, Cu, and Pb of 1 ppt or less was used. Furthermore, the proportions of quaternary ammonium ions and hydrogen ions contained in the counter ions of the strongly acidic ion-exchange resin after the pretreatment were measured to find that the proportion of quaternary ammonium ions was 80 mol% and the proportion of hydrogen ions was 20 mol%.

Through the column, 1050 L of the crude tetramethylammonium chloride aqueous solution was passed under a condition of SV = 20 (1/hr). Each time after the liquid flow amount reached 10 L (200 L/L-resin), 100 L (2000 L/L-resin), 500 L (10000 L/L-resin), and 1000 L (20000 L/L-resin), 10 L of the corresponding liquid was collected, and the contents of the metal impurities were measured. The results are each shown in Tables 7 and 8.

### Comparative Example 3

The same operation as in Example 1 was performed except for changing the strongly acidic ion-exchange resin to be used to Diaion UBK16 (degree of cross-linking 16, cation exchange capacity 2.3 equiv/L, Mitsubishi Chemical Corporation) in Comparative Example 2. The proportion of quaternary ammonium ions contained in the counter ions of the strongly acidic ion-exchange resin after the pretreatment at this time was 89 mol%, and the proportion of hydrogen ions was 11 mol%. The measurement results of the metal impurity contents are each shown in Tables 7 and 8.

### Comparative Example 4

The same operation as in Example 1 was performed except for changing the strongly acidic ion-exchange resin to be used to Diaion SK112 (degree of cross-linking 12, cation exchange capacity 2.1 equiv/L, available from Mitsubishi Chemical Corporation) in comparative Example 2. The proportion of quaternary ammonium ions contained in the counter ions of the strongly acidic ion-exchange resin after the pretreatment at this time was 97 mol%, and the proportion of hydrogen ions was 3 mol%. The measurement results of the metal impurity contents are each shown in Tables 7 and 8.

### Comparative Example 5

The same operation as in Example 1 was performed except for changing the strongly acidic ion-exchange resin to be used to Diaion SK1B (degree of cross-linking 8, cation exchange capacity 2.0 equiv/L, available from Mitsubishi Chemical Corporation) in Comparative Example 2. The proportion of quaternary ammonium ions contained in the counter ions of the strongly acidic ion-exchange resin after the pretreatment at this time was 99 mol%, and the proportion of hydrogen ions was 1 mol%. The measurement results of the metal impurity contents are each shown in Tables 7 and 8.

### Comparative Example 6

The same operation as in Example 1 was performed except for changing the strongly acidic ion-exchange resin to be used to Diaion PK212 (degree of cross-linking 6, cation exchange capacity 1.5 equiv/L, available from Mitsubishi Chemical Corporation) in Comparative Example 2. The proportion of quaternary ammonium ions contained in the counter ions of the strongly acidic ion-exchange resin after the pretreatment at this time was 99.9 mol% or more, and the proportion of hydrogen ions was 0.1 mol%. The measurement results of the metal impurity contents are each shown in Tables 7 and 8.

**[Table 7]**

| | | Metal (ppt) | | | | | | |
|---|---|---|---|---|---|---|---|---|
| | | Na | K | Li | Ca | Mg | Sr | Total |
| 60% Tetramethylammonium chloride aqueous solution (before purification) [content of large-molecular-weight organic impurity* 372 ppm] | | 3.6 x 10⁴ | 3.9 x 10³ | 5.1 x 10² | 4.5 x 10⁴ | 1.2 x 10³ | 1.1 x 10² | 8.7 x 10⁴ |
| Comparative Example 2 | 200 L/L-resin | 2 | < 1 | < 1 | < 1 | < 1 | < 1 | 7 |
| | 2000 L/L-resin | 2 | < 1 | < 1 | < 1 | < 1 | < 1 | 7 |
| | 10000 L/L-resin | 2 | < 1 | < 1 | < 1 | < 1 | < 1 | 7 |
| | 20000 L/L-resin | 4 | 3 | < 1 | < 1 | < 1 | < 1 | 11 |
| Comparative Example 3 | 200 L/L-resin | 2 | 2 | < 1 | < 1 | < 1 | < 1 | 8 |
| | 2000 L/L-resin | 2 | 2 | < 1 | < 1 | < 1 | < 1 | 8 |
| | 10000 L/L-resin | 3 | 4 | < 1 | < 1 | < 1 | < 1 | 11 |
| | 20000 L/L-resin | 8 | 7 | 2 | < 1 | < 1 | < 1 | 20 |
| Comparative Example 4 | 200 L/L-resin | 3 | 4 | < 1 | < 1 | < 1 | < 1 | 11 |
| | 2000 L/L-resin | 5 | 5 | < 1 | < 1 | < 1 | < 1 | 14 |
| | 10000 L/L-resin | 8 | 9 | 5 | < 1 | < 1 | < 1 | 25 |
| | 20000 L/L-resin | 3.6 x 10⁴ | 3.8 x 10³ | 5.1 x 10² | 4.4 x 10⁴ | 1.2 x 10³ | 1.1 x 10² | 8.6 x 10⁴ |
| Comparative Example 5 | 200 L/L-resin | 8 | 6 | < 1 | < 1 | < 1 | < 1 | 18 |
| | 2000 L/L-resin | 10 | 9 | < 1 | < 1 | < 1 | < 1 | 23 |
| | 10000 L/L-resin | 3.6 x 10⁴ | 3.9 x 10³ | 5.0 x 10² | 4.5 x 10⁴ | 1.2 x 10³ | 1.1 x 10² | 8.7 x 10⁴ |
| | 20000 L/L-resin | 3.6 x 10⁴ | 3.9 x 10³ | 5.1 x 10² | 4.5 x 10⁴ | 1.2 x 10³ | 1.1 x 10² | 8.7 x 10⁴ |
| Comparative Example 6 | 200 L/L-resin | 3.6 x 10⁴ | 3.8 x 10³ | 5.0 x 10² | 4.5 x 10⁴ | 1.2 x 10³ | 1.0 x 10² | 8.7 x 10⁴ |
| | 2000 L/L-resin | 3.6 x 10⁴ | 3.9 x 10³ | 5.1 x 10² | 4.5 x 10⁴ | 1.2 x 10³ | 1.1 x 10² | 8.7 x 10⁴ |
| | 10000 L/L-resin | 3.6 x 10⁴ | 3.9 x 10³ | 5.1 x 10² | 4.5 x 10⁴ | 1.2 x 10³ | 1.1 x 10² | 8.7 x 10⁴ |
| | 20000 L/L-resin | 3.6 x 10⁴ | 3.9 x 10³ | 5.1 x 10² | 4.5 x 10⁴ | 1.2 x 10³ | 1.1 x 10² | 8.7 x 10⁴ |

| | | | | | | | | |
|---|---|---|---|---|---|---|---|---|
| * Large-molecular-weight organic impurity: organic impurity with a molecular weight of 100 0 g/mol or more | | | | | | | | |

**[Table 8]**

| | | Metal (ppt) | | | | |
|---|---|---|---|---|---|---|
| | | Fe | Ni | Cu | Pb | Total |
| 60% Tetramethylammonium chloride aqueous solution (before purification) [content of large-molecular-weight organic impurity 372 ppm] | | 2.0 x 10³ | 1.5 x 10⁴ | 7.3 x 10² | 4.0 x 10 | 1.8 x 10⁴ |
| Comparative Example 2 | 200 L/L-resin | 2 | < 1 | 2 | < 1 | 6 |
| | 2000 L/L-resin | 2 | < 1 | 2 | < 1 | 6 |
| | 10000 L/L-resin | 3 | < 1 | 3 | < 1 | 8 |
| | 20000 L/L-resin | 4 | < 1 | 3 | < 1 | 9 |
| Comparative Example 3 | 200 L/L-resin | 2 | < 1 | 2 | < 1 | 6 |
| | 2000 L/L-resin | 3 | < 1 | 2 | < 1 | 7 |
| | 10000 L/L-resin | 3 | < 1 | 4 | < 1 | 9 |
| | 20000 L/L-resin | 5 | 2 | 5 | < 1 | 13 |
| Comparative Example 4 | 200 L/L-resin | 3 | < 1 | 3 | < 1 | 8 |
| | 2000 L/L-resin | 4 | < 1 | 3 | < 1 | 9 |
| | 10000 L/L-resin | 7 | < 1 | 5 | < 1 | 14 |
| | 20000 L/L-resin | 1.9 x 10³ | 1.3 x 10⁴ | 7.2 x 10² | 3.7 x 10 | 1.6 x 10⁴ |
| Comparative Example 5 | 200 L/L-resin | 3 | < 1 | 3 | < 1 | 8 |
| | 2000 L/L-resin | 8 | < 1 | 5 | < 1 | 15 |
| | 10000 L/L-resin | 2.0 x 10³ | 1.5 x 10⁴ | 7.2 x 10² | 3.8 x 10 | 1.8 x 10⁴ |
| | 20000 L/L-resin | 2.0 x 10³ | 1.5 x 10⁴ | 7.2 x 10² | 3.9 x 10 | 1.8 x 10⁴ |
| Comparative Example 6 | 200 L/L-resin | 2.0 x 10³ | 1.5 x 10⁴ | 7.2 x 10² | 3.8 x 10 | 1.8 x 10⁴ |
| | 2000 L/L-resin | 2.0 x 10³ | 1.5 x 10⁴ | 7.3 x 10² | 4.0 x 10 | 1.8 x 10⁴ |
| | 10000 L/L-resin | 2.0 x 10³ | 1.5 x 10⁴ | 7.3 x 10² | 4.0 x 10 | 1.8 x 10⁴ |
| | 20000 L/L-resin | 2.0 x 10³ | 1.5 x 10⁴ | 7.3 x 10² | 4.0 x 10 | 1.8 x 10⁴ |

### Comparative Examples 7 to 11

For 25 mass% crude tetramethylammonium hydroxide aqueous solutions, the metal impurity contents were measured. The results are each shown in Tables 9 and 10. In addition, the content of an organic impurity with a molecular weight of 1000 g/mol or more was also measured and found to be 43 ppm.

The same operations as in Comparative Examples 2 to 6 were performed except for using the crude tetramethylammonium hydroxide aqueous solutions thus prepared (Comparative Example 7 corresponds to Comparative Example 2, Comparative Example 8 corresponds to Comparative Example 3, Comparative Example 9 corresponds to Comparative Example 4, Comparative Example 10 corresponds to Comparative Example 5, and Comparative Example 11 corresponds to Comparative Example 6). Each measurement result of the metal impurity contents is shown each in Tables 9 and 10 above.

**[Table 9]**

| | | Metal (ppt) | | | | | | |
|---|---|---|---|---|---|---|---|---|
| | | Na | K | Li | Ca | Mg | Sr | Total |
| 25% Tetramethylammonium hydroxide aqueous solution (before purification) [content of large-molecular-weight organic impurity 43 ppm] | | 37 | 25 | 3 | 41 | 3 | 2 | 111 |
| Comparative Example 7 | 200 L/L-resin | 1 | 1 | < 1 | 1 | < 1 | < 1 | 6 |
| | 2000 L/L-resin | 1 | 1 | < 1 | 1 | < 1 | < 1 | 6 |
| | 10000 L/L-resin | 2 | 1 | < 1 | 2 | < 1 | < 1 | 8 |
| | 20000 L/L-resin | 3 | 2 | < 1 | 3 | < 1 | < 1 | 11 |
| Comparative Example 8 | 200 L/L-resin | 1 | 1 | < 1 | 1 | < 1 | < 1 | 6 |
| | 2000 L/L-resin | 1 | 1 | < 1 | 1 | < 1 | < 1 | 6 |
| | 10000 L/L-resin | 2 | 2 | < 1 | 3 | < 1 | < 1 | 10 |
| | 20000 L/L-resin | 5 | 3 | < 1 | 4 | < 1 | < 1 | 15 |
| Comparative Example 9 | 200 L/L-resin | 1 | 1 | < 1 | 2 | < 1 | < 1 | 7 |
| | 2000 L/L-resin | 2 | 1 | < 1 | 2 | < 1 | < 1 | 8 |
| | 10000 L/L-resin | 4 | 2 | < 1 | 4 | < 1 | < 1 | 13 |
| | 20000 L/L-resin | 6 | 4 | 1 | 6 | 1 | < 1 | 19 |
| Comparative Example 10 | 200 L/L-resin | 1 | 2 | < 1 | 2 | < 1 | < 1 | 8 |
| | 2000 L/L-resin | 2 | 2 | 1 | 3 | < 1 | < 1 | 10 |
| | 10000 L/L-resin | 5 | 4 | 1 | 6 | 1 | < 1 | 18 |
| | 20000 L/L-resin | 8 | 7 | 1 | 8 | 1 | < 1 | 26 |
| Comparative Example 11 | 200 L/L-resin | 33 | 23 | 3 | 38 | 3 | 2 | 102 |
| | 2000 L/L-resin | 34 | 24 | 3 | 40 | 3 | 2 | 106 |
| | 10000 L/L-resin | 36 | 25 | 3 | 40 | 3 | 2 | 109 |
| | 20000 L/L-resin | 36 | 25 | 3 | 41 | 3 | 2 | 110 |

**[Table 10]**

| | | Metal (ppt) | | | | |
|---|---|---|---|---|---|---|
| | | Fe | Ni | Cu | Pb | Total |
| 25% Tetramethylammonium hydroxide aqueous solution (before purification) [content of large-molecular-weight organic impurity 43 ppm] | | 5 | 7 | 3 | 3 | 18 |
| Comparative Example 7 | 200 L/L-resin | 4 | 4 | 2 | 2 | 12 |
| | 2000 L/L-resin | 4 | 4 | 2 | 2 | 12 |
| | 10000 L/L-resin | 4 | 5 | 2 | 2 | 13 |
| | 20000 L/L-resin | 4 | 5 | 3 | 2 | 14 |
| Comparative Example 8 | 200 L/L-resin | 4 | 4 | 2 | 2 | 12 |
| | 2000 L/L-resin | 4 | 4 | 2 | 2 | 12 |
| | 10000 L/L-resin | 4 | 5 | 3 | 2 | 14 |
| | 20000 L/L-resin | 4 | 5 | 3 | 3 | 15 |
| Comparative Example 9 | 200 L/L-resin | 4 | 4 | 2 | 2 | 12 |
| | 2000 L/L-resin | 4 | 5 | 2 | 2 | 13 |
| | 10000 L/L-resin | 4 | 5 | 3 | 3 | 15 |
| | 20000 L/L-resin | 4 | 6 | 3 | 3 | 16 |
| Comparative Example 10 | 200 L/L-resin | 4 | 5 | 2 | 3 | 14 |
| | 2000 L/L-resin | 4 | 5 | 3 | 3 | 15 |
| | 10000 L/L-resin | 4 | 6 | 3 | 3 | 16 |
| | 20000 L/L-resin | 4 | 6 | 3 | 3 | 16 |
| Comparative Example 11 | 200 L/L-resin | 5 | 6 | 3 | 3 | 17 |
| | 2000 L/L-resin | 5 | 7 | 3 | 3 | 18 |
| | 10000 L/L-resin | 5 | 7 | 3 | 3 | 18 |
| | 20000 L/L-resin | 5 | 7 | 3 | 3 | 18 |

In Examples 1 to 10, in which the crude quaternary ammonium aqueous solutions in which content of an organic impurity with a molecular weight of 1000 g/mol or more was 1000 ppm or less and the quaternary ammonium ion was an ethyltrimethylammonium ion were treated, an excellent effect of decreasing the metal impurity concentrations was exhibited in all cases at the initial stage of the treatment when the liquid flow amount was 10 L (200 L/L-resin) by the contact with the cation-exchange resin with a degree of cross-linking of 6 or more in which a counter ion was of a non-metal ion type. In addition, in comparison with Comparative Examples 2 to 11, in which the crude quaternary ammonium aqueous solutions in which the quaternary ammonium ion was a tetramethylammonium ion were treated, the metal impurities were able to be removed also in the case of lower cross-linking, and the removal effect was maintained also in the case of larger liquid flow amounts.

Furthermore, in Comparative Example 1, in which the amount of an organic impurity with a molecular weight of 1000 g/mol or more contained in the crude quaternary ammonium aqueous solution to be treated exceeded 1000 ppm, the metal impurities were able to be removed to some extent at the initial stage of the treatment when the liquid flow amount was 10 L (200 L/L-resin), but the effect resulted in a rapid decrease when the liquid flow amount was continued to 100 L (2000 L/L-resin).

### Reference Signs List

1: Anode
2: Cathode
3: Power source
4: Anode chamber
5: Raw material chamber
6: Intermediate chamber
7: Cathode chamber
8: Anion-exchange membrane
9: Cation-exchange membrane

## Claims

1. A method for producing a purified quaternary ammonium compound aqueous solution, the method comprising bringing a crude quaternary ammonium compound aqueous solution into contact with a cation-exchange resin with a degree of cross-linking of 6 or more in which a counter ion is a non-metal ion type,
wherein
the quaternary ammonium compound aqueous solution comprises
1000 ppm or less of an organic impurity with a molecular weight of 1000 g/mol or more,
a quaternary ammonium ion represented by Formula (1), and
a metal impurity, 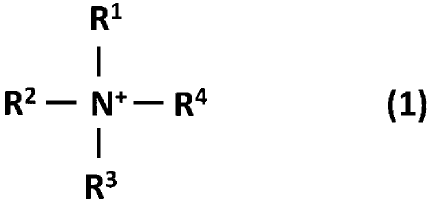
where R¹, R², R³, and R⁴ are each independently an alkyl group having carbon number from 1 to 16, with the proviso that the carbon number of one or more alkyl groups of R¹, R², R³, and R⁴ is from 2 to 16.

2. The method for producing a purified quaternary ammonium compound aqueous solution according to claim 1, wherein all of R¹, R², R³, and R⁴ in Formula (1) are not all the same alkyl group.

3. The method for producing a purified quaternary ammonium compound aqueous solution according to claim 1, wherein three of R¹, R², R³, and R⁴ in Formula (1) are identical groups, and the remaining one is an alkyl group having carbon number from 2 to 16 and different from the three identical groups.

4. The method for producing a purified quaternary ammonium compound aqueous solution according to any one of claims 1 to 3, wherein the cation-exchange resin has a degree of cross-linking of 8 to 30.

5. The method for producing a purified quaternary ammonium compound aqueous solution according to any one of claims 1 to 4, wherein the cation-exchange resin comprises a strongly acidic cation-exchange resin.

6. The method for producing a purified quaternary ammonium compound aqueous solution according to any one of claims 1 to 5, wherein the cation-exchange resin is brought into contact with the crude quaternary ammonium compound aqueous solution by a liquid flow method in a treatment amount of the crude quaternary ammonium compound aqueous solution to the cation-exchange resin of 2000 (L/L-resin) under a condition of a space velocity SV = 1 to 20 (1/hr).

7. The method for producing a purified quaternary ammonium compound aqueous solution according to any one of claims 1 to 6, wherein the cation-exchange resin is produced by bringing a cation-exchange resin in which a counter ion is a hydrogen ion type into contact with a quaternary ammonium compound aqueous solution comprising a quaternary ammonium ion identical to that of the crude quaternary ammonium compound aqueous solution.

8. The method for producing a purified quaternary ammonium compound aqueous solution according to any one of claims 1 to 7, wherein the crude quaternary ammonium compound aqueous solution is a crude quaternary ammonium hydroxide compound aqueous solution, a crude quaternary ammonium halide compound aqueous solution, or a crude quaternary ammonium carbonate compound aqueous solution.
